# EUROPÄISCHE PATENTSCHRIFT

(11) **EP 0 146 080 B1**
(45) Veröffentlichungstag und Bekanntmachung des Hinweises auf die Patenterteilung: **26.07.1989**
(21) Anmeldenummer: 84114820.8
(22) Anmeldetag: 06.12.1984
(51) Int. Cl.: C07D 209/52, C07B 57/00

(54) **Verfahren zur Racematspaltung bicyclischer Imino-alpha-carbonsäureester**
Process for the racemic separation of bicyclic alpha-imino carboxylic-acid esters
Procédé de séparation racémique d'esters bicycliques d'amides alpha-imino-carboxyliques

(30) Priorität: 15.12.1983 DE 3345355
(43) Veröffentlichungstag der Anmeldung: 26.06.1985
(73) Patentinhaber: HOECHST AKTIENGESELLSCHAFT, 65926 Frankfurt am Main (DE)
(72) Erfinder: Ulrich, Lerch, Dr., D-6238 Hofheim am Taunus (DE)

(56) Entgegenhaltungen:
- EP-A- 0 115 345

## Beschreibung

Die saubere und quantitative Auftrennung eines Racemats mittels Kristallisation eines diastereomeren Salzes ist ein Wunschziel, das selten einmal erreicht wird. Normalerweise sind umfangreiche fraktionierte Kristallisationen erforderlich, die, abgesehen vom hohen Arbeitsaufwand, oft mit großen Substanzverlusten verbunden sind. Voraussagen, welche speziellen Bedingungen für eine erfolgreiche Racematspaltung im Einzelfall zu wählen sind, lassen sich a priori nicht machen, so daß die Frage nach dem optisch aktiven Hilfstoff und dem geeigneten Lösungsmittel jedesmal offen bleibt (vgl. hierzu J.P. Greenstein, M. Winitz, «Chemistry of the Amino Acids», New York (1961), Seiten 716 und 717).

Ein Verfahren zur Racemattrennung bicyclischer Imino-a-carbonsäureester ist bereits vorgeschlagen worden (EP-A-115345). Dabei werden diastereomere Salze dieser Verbindungen mit N-acylierten, optisch aktiven Aminocarbonsäuren, wie z.B. N-Benzyloxycarbonyl-S-phenylalanin, hergestellt und durch fraktionierte Kristallisation getrennt.

Überraschenderweise wurde gefunden, daß sich auch O,O-Diacylweinsäuren hervorragend zur Trennung von bicyclischen Imino-α-carbonsäuren in die optischen Antipoden eignen.

Die Erfindung betrifft somit ein Verfahren zur Auftrennung racemischer Gemische bicyclischer Imino-α-carbonsäureester in die Komponenten der Formel la und Ib 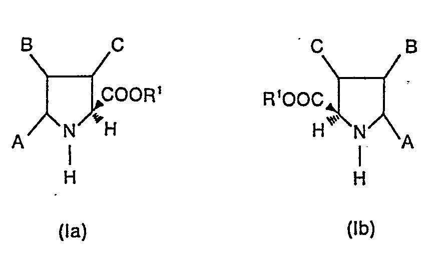 in welcher
R¹ für Alkyl mit 1 bis 6 C-Atomen, vorzugsweise Benzyl tert.-Butyl, Cycloalkyl mit 4 bis 8 C-Atomen oder Aralkyl mit 7 bis 13 C-Atomen, vorzugsweise Benzyl, steht,
a) A Wasserstoff bedeutet und
B und C gemeinsam eine Kette der Formel -[CH₂]ₙ- mit n = 3, 4, 5 oder 6 oder eine Kette der Formel -[CH₂]ₚ-CH = CH-[CH₂]_{q}- mit (p + q) = 1, 2, 3 oder 4 bilden oder
b) C Wasserstoff bedeutet und
A und B gemeinsam eine der vorstehend unter a) definierten Ketten bilden
durch Kristallisation djastereomerer Salze, das dadurch gekennzeichnet ist, daß man die Salze der racemischen Ester der Formel la und Ib mit. optisch aktiven O,O-Diacylweinsäuren der Formel II 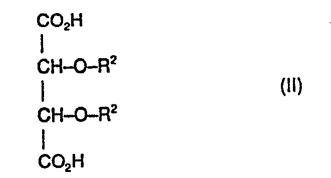 worin R² (C₁-C₆)-Alkanoyl oder (C₇-C₁₁)-Aroyl, vorzugsweise Benzoyl bedeutet, in einem Ester, einem Keton, einem Ether, einem niedrigen Alkohol, einem Nitril oder einem halogenierten Kohlenwasserstoff, oder einem Lösungsmittelgemisch, das eines oder mehrere Lösungsmittel aus den aufgezählten Verbindungsklassen enthält, herstellt, eines der beiden diastereomeren Salze kristallisiert, dieses diastereomere Salz ggfs. durch Umkristallisieren, Umlösen oder Ausrühren reinigt und schließlich durch Zusatz von Base in die reinen Enantiomeren der Formeln la bzw. Ib zerlegt und diese direkt weiterverwendet oder durch Salzbindung mit Mineralsäure in eine lagerfähige Form bringt.

Aus der Mutterlauge, die nach Abtrennen des schwerer löslichen diastereomeren Salzes erhalten wird, läßt sich das leichter lösliche diastereomere Salz und damit der zweite Antipode von I isolieren.

Bevorzugt werden solche racemischen Gemische von Estern der Formel la und Ib aufgetrennt, in welchen die beiden Brückenkopf-Wasserstoffatome cis-konfiguriert sind und die COOR-Gruppe endo-ständig zum bicyclischen Ringsystem orientiert sind und/oder in welchen A und B -[CH₂]₃-oder -[CH₂]₄- und C Wasserstoff bedeuten.

Die optisch einheitlichen bicyclischen Imino-a-carbonsäureester können beispielsweise als Ausgangsmaterialien zur Synthese optisch reiner Angiotensin-Converting-Enzym-Inhibitoren dienen. Verbindungen dieses Typs sind beispielsweise aus der EP-A-50 800, der EP-A-49 658, der EP-A-46 953, der EP-A-79022 und dem US-Patent 4 344 949 bekannt; sie können zur Bekämpfung des Bluthochdrucks eingesetzt werden.

Gegenüber dem in der EP-A-115345 vorgeschlagenen Verfahren hat das erfindungsgemäße Verfahren den Vorteil, daß die als Hilfsstoff verwendeten Diacylweinsäuren wesentlich wirtschaftlicher sind als die dort notwendigen N-AcylDerivate optisch aktiver R- oder S-Aminosäuren, ein Umkristallisieren nicht erforderlich ist und die Ausbeuten an optisch einheitlichen Antipoden la bzw. Ib höher sind. Auch der chemische Reinheitsgrad der nach dem erfindungsgemäßen Verfahren erhaltenen Antipoden la bzw. Ib ist höher.

Die racemischen Verbindungen der Formel la + Ib werden zunächst aus ihren Salzen mit anorganischen Säuren freigesetzt. Dazu verwendet man ein Zweiphasensystem aus verdünnter Natronlauge und vorzugsweise einen Ether wie beispielsweise Diisopropylether oder tert.-Butylmethylether. Man rührt das Salz in diesem Gemisch vorzugsweise bei Temperaturen zwischen 0° und 20°C und trennt die Phasen, bis sich alles gelöst hat. Nach dem Waschen der organischen Phase mit Wasser und Extraktion der wäßrigen Phase mit dem entsprechenden Ether dampft man die organische Phase ein und erhält die freie racemische Verbindung I in guter Reinheit und hoher Ausbeute (96-99%). Außerdem hat man bei der Phasentrennung keine Probleme durch Emulsionsbildung, wie dies beispielsweise bei Verwendung von Methylenchlorid der Fall ist.

Die diastereomeren Diacyltartrate werden hergestellt durch Reaktion der racemischen bicyclischen Imino-a-carbonsäureester der Formel la + Ib mit optisch aktiver O,O-Diacylweinsäure der Formel II. Dabei werden 0,5 bis 1,5 Mol der Diacylweinsäure, vorzugsweise 0,9-1,1 Mol verwendet.

Als Lösungsmittel für die Bildung der diastereomeren Diacyltartrate werden solche bevorzugt, aus denen eines der beiden diastereomeren Salze auskristallisiert, während das zweite in Lösung bleibt. Bevorzugt hierfür sind Ester, vorzugsweise niedere Ester wie Ethylacetat, Ketone wie Aceton oder 2-Butanon, Ether wie Tetrahydrofuran, 1,2-Dimethoxyethan oder Dioxan, niedrigmolekulare Alkohole wie Methanol, Ethanol oder Isopropanol, Nitrile wie Acetonitril, halogenierte Kohlenwasserstoffe wie z.B. Methylenchlorid. Diese Lösungsmittel, insbesondere Ethylacetat, können allein verwendet werden, bevorzugt sind jedoch Gemische, die eines oder mehrere Lösungsmittel aus der Reihe der oben aufgezählten Verbindungsklassen enthalten, insbesondere Gemische mit solchen wenig polaren Lösungsmittel, in denen die Diacyltartrate von Verbindungen der Formel I wenig löslich sind, z.B. höhere Ester wie Butylacetat, Aromaten wie Toluol, polare Ether wie Diethylether, Diisopropylether oder tert.-Butylmethylether, Kohlenwasserstoffe wie Cyclohexan, Hexan oder Petrolether.

Besonders bevorzugt als Lösungsmittel für die Bildung der diastereomeren Salze aus la bzw. Ib und II sind Ethylacetat, Aceton und 2-Butanon, ggfs. zusammen mit weniger polaren Lösungsmit-' tein wie tert.-Butylmethylether, Diisopropylether, Toluol oder Butylacetat. Als Reaktionstemperaturen kommen Temperaturen von -20 bis +50°C in Frage. Bevorzugt sind jedoch Temperaturen von -5 bis +30°C.

In einer bevorzugten Ausführungsform des erfindungsgemäßen Verfahrens wird eine Lösung von O,O-Dibenzoyl-L-weinsäure in Aceton, Ethylacetat oder 2-Butanon mit dem Ester der racemischen bicyclischen Imino-α-carbonsäure der Formeln la und Ib versetzt, ggfs. mit dem Produkt angeimpft und danach eine genau bestimmte Menge von z. B. tert.-Butylmethylether nach einem definierten Dosierungsschema zugegeben. Man rührt zunächst bei Raumtemperatur und kühlt danach auf Temperaturen zwischen -5 und +5°C ab. Nach dem Entfernen der Mutterlauge und gründlichem Waschen erhält man das diastereomere Salz in Ausbeuten von 80-100%.

Die Erfindung betrifft auch diastereomere Salze aus bicyclischen Imino-a-carbonsäureestern der Formel la oder Ib mit einer optisch aktiven O,O-Diacylweinsäure der Formel II.

Die isolierten diastereomeren Salze fallen nach dem erfindungsgemäßen Verfahren im allgemeinen so optisch rein an, daß sie ohne einen weiteren Reinigungsschritt direkt zerlegt werden können.

Sofern eine weitere Reinigung des Salzes notwendig ist, kann sie durch Umkristallisieren, Umfällen oder Ausrühren erfolgen. Im wesentlichen verwendet man dabei die gleichen oder ähnliche Lösungsmittel bzw. Lösungsgemische, wie sie zur Herstellung der diastereomeren Salze verwendet wurden.

Beispielsweise kann man zum Umkristallisieren in heißem Aceton oder 2-Butanon lösen, unter Rühren und Animpfen erkalten lassen, ggfs. unter Zugabe eines unpolaren Lösungsmittels wie z. B. tert.-Butylmethylether, Diisopropylether oder Butylacetat, kühlen und das gereinigte Salz durch Filtration isolieren.

Eine andere Möglichkeit (Umfällen) besteht darin, ohne thermische Belastung des diastereomeren Salzes bei Raumtemperatur in einer kleinen Menge eines Lösungsmittels zu lösen, in dem das Salz gut löslich ist, wie z. B. in einem niedrigmolekularen Alkohol und danach durch Zusatz eines weniger polaren Lösungsmittels oder Lösungsmittelgemisches, wie z.B. Ethylacetatltert.-Butylmethylether, das Salz zur Kristallisation zu bringen.

Schließlich ist es auch möglich (Ausrühren), das rohe diastereomere Salz mit einem geeignetem Lösungsmittel wie z.B. Ethylacetat oder einem Lösungsmittelgemisch wie z.B. 2-Butanon/ tert.Butylmethylether bei Raumtemperatur als Suspension zu rühren, danach abzukühlen und zu filtrieren.

Zur Zerlegung der optisch reinen diastereomeren Salze wird mit einer Base in Wasser versetzt und die optisch aktive Verbindung la bzw. Ib in einem mit Wasser nicht mischbaren Lösungsmittel aufgenommen.

Als Basen sind wäßrige Alkalihydroxyde wie Natrium-, Kalium- oder Lithiumhydroxyd besonders gut geeignet.

Als organische Lösungsmittel werden nach dem erfindungsgemäßen Verfahren solche verwendet, aus denen man durch Zugabe einer anorganischen Säure direkt das mineralsaure Salz der optisch reinen Verbindung la bzw. Ib ausfällen und durch Filtration isolieren kann. Dazu eignen sich z. B. Ether wie Diisopropylether oder tert. Butylmethylether, Kohlenwasserstoffe wie Petrolether oder Toluol, Methylenchlorid oder Ethylacetat.

Die Zerlegung des Salzes wird vorzugsweise bei - bis +30°C vorgenommen; besonders bevorzugt sind Temperaturen zwischen 0 und 20 °C, um die Verseifung der Esterfunktion zu unterdrükken.

Im einzelnen geht man bei der Zerlegung des optisch reinen Salzes bevorzugt wie folgt vor: Das Salz wird in einem der obengenannten Lösungsmittel, vorzugsweise in Diisopropylether, tert. Butylmethylether oder Toluol suspendiert und unter Rühren bei 0 bis 20°C eine Lösung der 2-3 molaren Menge Natriumhydroxyd in Wasser zugegeben. Man rührt, bis sich die Kristalle vollständig gelöst haben und trennt die Phasen. Der optisch reine Imino-a-carbonsäureester der Formel la bzw. Ib kann durch Verdampfen des organischen Lösungsmittels isoliert werden oder vorteilhafterweise durch Zusatz einer anorganischen Säure, wie z. B. gasförmigem oder etherischem Chlorwasserstoff oder Schwefelsäure, als Salz ausgefällt werden. In Salzform sind Verbindungen der Formel la bzw. Ib lagerstabil. Nach dem erfindungsgemäßen Verfahren werden die mineralsauren Salze der optisch reinen Verbindungen la bzw. Ib in Ausbeuten von 75-90% bezogen auf die als Ausgangsmaterial verwendeten Hydrochloride der racemischen Verbindungen erhalten. Die Produkte zeichnen sich durch große chemische und optische Reinheit aus.

### Beispiel 1

### A) Racem. Cis-endo-2-Azabicyclo[3.3.0]octan-3-carbonsäurebenzylester

56,4 g (0,2 Mol) racemisches cis-endo-2-Azabi- cyclo[3.3.0]octan-3-carbonsäurebenzylester-hydrochlorid werden in 250 ml tert. Butylmethylether suspendiert und bei 0-5°C Innentemperatur unter kräftigem Rühren eine Lösung von 8,8 g (0,22 Mol) NaOH in 90 ml Wasser zugegeben. Sobald alles in Lösung gegangen ist (nach etwa 15 Minuten), werden die Phasen getrennt. Die wäßrige Phase wird mit je zweimal 30 mi tert. Butylmethylether extrahiert und verworfen. Die vereinigten organischen Phasen wäscht man je zweimal 30 ml Wasser und trocknet über wasserfreiem Natriumsulfat. Nach Einengen i. Vak. und zuletzt i. Hochvak. erhält man 47,5-48,5 g eines fast farblosen Öls (96,8-98,8%).

### B) Dibenzoyltartrat des cis-endo-Azabicyclo-[3.3.0]octan-3-(S)-carbonsäurebenzylesters

37,7 g (0,1 Mol) Dibenzoyl-L-weinsäurehydrat werden bei Raumtemperatur in 60 ml 2-Butanon gelöst. Dazu gibt man bei Innentemperatur von 10-15 °C 24,6 g (0,1 Mol) des racem. cis-endo-2-Azabicyclo[3.3.0]octan-3-carbonsäurebenzyl- esters in 40 ml 2-Butanon (exotherme Reaktion) und impft mit etwa 0,2 g des Dibenzoyltartrats an. Man rührt 30 Minuten kräftig bei der oben angegebenen Temperatur, bis ein dicker, farbloser Brei entstanden ist (etwa 30 Minuten). Nun wird auf 0 °C Innentemperatur gekühlt, 30 Minuten bei dieser Temperatur gerührt und danach eine Mischung von 100 ml tert. Butylmethylether und 25 ml 2-Butanon innerhalb von 30 Minuten zulaufen gelassen, wobei eine Innentemperatur von 0°C aufrechterhalten wird. Man rührt noch 1 Stunde bei dieser Temperatur nach, saugt ab und wäscht sorgfältig mit zweimal 20 ml einer 1:1-Mischung von tert. Butylmethylether und 2-Butanon. Nach dem Trocknen erhält man 27,0 g (89,5% = 88,3% bezogen auf das racemische Hydrochlorid) des gewünschten Salzes vom Fp. 108-109 °C, [α]²⁰_{D} = -87,7° (c = 1, CH₃OH). C) Cis-endo-2-Azabicyclo[3.3.0]octan-3-(S)-carbonsäurebenzylesterhydrochlorid

18,11 g (30 mMol) des nach Beispiel 1B hergestellten, ungereinigten Dibenzoyltartrats werden mit75 ml Diisopropylether intensiv gerührt, bis die Kristallbrocken weitgehend zerfallen sind und ein glatter Brei entstanden ist. Bei Temperaturen unter 15°C gibt man eine Lösung von 3,0 g (75 mMol) NaOH in 50 ml Wasser zur Suspension und rührt intensiv, bis praktisch alles gelöst ist. Die Phasen werden getrennt, die wäßrige Phase wird mit zweimal 10 ml Diisopropylether extrahiert und die vereinigten organischen Phasen mit zweimal 10 ml Wasser gewaschen, über Natriumsulfat getrocknet, mit etwas Hydrochlorid des optisch aktiven Aoc-Benzylesters angeimpft und unter Rühren ätherische Salzsäure langsam bis zur deutlich sauren Reaktion zugegeben. Das farblose Hydrochlorid wird nach 10-minütigem Rühren abgesaugt und mit zweimal 10 ml Diisopropylether gewaschen. Nach dem Trocknen erhält man 8,1 g (95,8%) farblose Kristalle.
Fp. 183-185 °C. 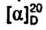
= -32,7° (c = 1, CHₐOH) -38,4° (c = 1, H₂O)

Gesamtausbeute, bezogen auf eingesetztes Hydrochlorid des racem. cis-endo-2-Azabicy- clo[3.3.0]octan-3-carbonsäurebenzylesters: 84,6%.

### Beispiel 2

### A. Dibenzoyltartrat des (S)-Azabicyclo[3.3.0]-octan-3-carbonsäurebenzylesters

1. 15,1 g (0,04 Mol) Dibenzoyl-L-weinsäurehydrat werden bei Raumtemperatur in 50 ml Ethylacetat gelöst und bei 15-20°C Innentemperatur 9,82 g (0,04 Mol) des racemischen cis-endo-2-Azabicyclo[3.3.0]octan-3-carbonsäurebenzyl- esters zugegeben. Man impft mit etwa 0,1 g des Dibenzoyltartrats an, rührt bei der oben angegebenen Temperatur 15-30 Minuten, bis ein dicker Brei entstanden ist und kühlt dann auf 0°C Innentemperatur ab. 15 Minuten nach Erreichen dieser Temperatur läßt man langsam (innerhalb von 15 Minuten) eine Mischung von 40 ml Ethylacetat und 8 ml tert. Butylmethylether zutropfen. Danach rührt man noch eine Stunde bei 0 °C und saugt die schwach gelb gefärbte Suspension ab, wäscht zweimal mit einer Mischung von je 9 ml Ethylacetat und 1 ml tert. Butylmethylether und trocknet bei Raumtemperatur. Man erhält 12,1 g (100%) leicht gelbstichige Kristalle vom Fp.104-108°C 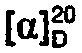 = -84,7° (c = 1, CH₃OH).
2. 10 g des erhaltenen rohen Dibenzoyltartrats werden 30 Minuten in 30 ml 2-Butanon bei Raumtemperatur gerührt. Man gibt dann innerhalb von 15 Minuten 30 ml tert. Butylmethyläther dazu, rührt 1 Stunde bei Raumtemperatur, danach 1 Stunde bei 0°C. Man saugt ab und wäscht mit zweimal 8 ml 2-Butanon/tert. Butylmethylether 4:6. Nach dem Trocknen erhält man 8,7 g farblose Kristalle (87% d. Th. bezogen auf den racem. cis- end-2-Azabicyclo[3.3.0]octan-3-carbonsäureben- zylester).
   Fp. 106-108°, 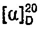 = -88,3° (c = 1, CH₃OH). B) Cis-endo-2-Azabicyclo[3.3.0]octan-3-(S)-carbonsäurebenzylesterhydrochlorid

Aus dem nach Beispiel 2 A2 hergestellten Dibenzoyltartrat wurde nach der im Beispiel 1c beschriebenen Methode das Hydrochlorid des cis- endo-2-Azabicyclo[3.3.0]octan-3-(S)-carbonsäure- benzylesters hergestellt. Fp. 184-185,5 °C 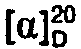 = -32,5° (c = 1, CH₃0H)
- 37,1° (c = 1, H₂0)

Gesamtausbeute, bezogen auf das eingesetzte racem. Hydrochlorid 79,2% d. Th.

### Beispiel 3

### A) Umkristallisieren des rohen Dibenzoyltartrats des cis-endo-2-Azabicyclo[3.3.0]octan-3-(S)-carbonsäurebenzylesters

15 g des nach Beispiel 1 B hergestellten rohen Dibenzoyltartrats werden in 45 ml 2-Butanon, das auf 60-65°C vorgeheizt wurde, rasch gelöst und ohne weitere Heizung sofort 40 ml tert. Butylmethylether zugegeben, wobei die Temperatur auf etwa 35-40 °C sinken soll. Man impft an und kühlt auf 20°C, läßt 30 Minuten bei dieser Temperatur rühren und gibt dann weitere 40 ml tert. Butylmethylether innerhalb von 10 Minuten zu und rührt schließlich 1 Stunde bei 0°C. Man erhält so 13,4 g (89,3%) farblose Kristalle vom Fp. 110-112 °C [α]²⁰_{D} = -87,1° (c = 1, CH₃0H).

### B) Cis-endo-2-Azabicyclo[3.3.0]octan-3-(S)-carbonsäurebenzylesterhydrochlorid

Auf die gleiche Weise wie im Beispiel 1 C beschrieben, wurde aus dem umgelösten Dibenzoyltartrat 93,7% d. Th. Hydrochlorid gewonnen. Fₚ. 191-192 °C
[α]²⁰_{D} = -33,8° (c = 1, CH₃0H) -39,6° (c = 1, H₂0)

Gesamtausbeute, bezogen auf das eingesetzte rac. Hydrochlorid = 73,3%.

## Patentansprüche (Patentansprüche für folgende(n) Vertragsstaat(en) : BE, CH, DE, FR, GB, IT, LI, LU, NL, SE)

1. Verfahren zur Auftrennung racemischer Gemische bicyclischer Imino-a-carbonsäureester in die Komponenten der Formel la und Ib 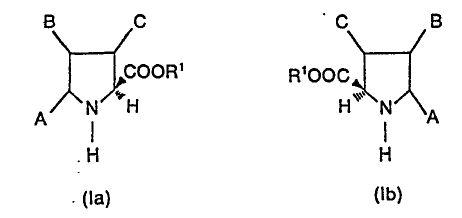 in welcher
R' für Alkyl mit 1 bis 6 C-Atomen, Cycloalkyl mit 4 bis 8 C-Atomen oder Aralkyl mit 7 bis 13 C-Atomen steht,
a) A Wasserstoff bedeutet und
B und C gemeinsam eine Kette der Formel -[CH₂]ₙ- mit n = 3, 4, 5 oder 6 oder eine Kette der Formel -[CH₂]p-CH=CH-[CH₂]_{q} mit (p + q) = 1, 2, 3 oder 4 bilden oder
b) C Wasserstoff bedeutet und
A und B gemeinsam eine der vorstehend unter a) definierten Ketten bilden durch Kristallisation diastereomerer Salze, dadurch gekennzeichnet, daß man die Salze der racemischen Ester der Formel la und Ib mit optisch aktiven O,O-Diacylweinsäuren der Formel II 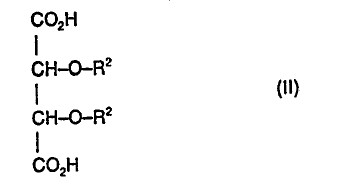 worin R² (C₁-C₆)-Alkanoyl oder (C₇-C₁₁)-Aroyl bedeutet, in einem Ester, einem Keton, einem Ether, einem niedrigen Alkohol, einem Nitril oder einem halogenierten Kohlenwasserstoff, oder einem Lösungsmittelgemisch, das eines oder mehrere Lösungsmittel aus den aufgezählten Verbindungsklassen enthält, herstellt, eines der beiden diastereomeren Salze kristallisiert, dieses diastereomere Salz ggf. durch Umkristallisieren, Umlösen oder Ausrühren reinigt und schließlich durch Zusatz von Base in die reinen Enantiomeren der Formeln la bzw. Ib zerlegt und diese direkt weiterverwendet oder durch Salzbildung mit Mineralsäure in eine lagerfähige Form bringt.

2. Verfahren nach Anspruch 1, dadurch gekennzeichnet, daß man die Salze von Estern der Formeln la bzw. Ib, in welchen die beiden Brückenkopf-Wasserstoffatome cis-konfiguriert sind und die COOR-Gruppe endo-ständig zum bicyclischen Ringsystem orientiert sind, mit Säuren der Formel 11 herstellt und kristallisiert.

3. Verfahren nach Anspruch 1 oder 2, dadurch gekennzeichnet, daß A und B gemeinsam für -[CH₂]ₙ- mit n = 3 oder 4 stehen und C Wasserstoff bedeutet.

4. Verfahren gemäß einem der Ansprüche 1 bis 3, dadurch gekennzeichnet, daß man die diastereomeren Salze in einem Ester, einem Keton, einem Ether, einem niedrigen Alkohol, einem Nitril oder einem halogenierten Kohlenwasserstoff herstellt.

5. Verfahren gemäß einem der Ansprüche 1 bis 4, dadurch gekennzeichnet, daß man die diastereomeren Salze in einem Lösungsmittelgemisch herstellt, das eines oder mehrere Lösungsmittel aus der Reihe der im Anspruch 4 aufgezählten Verbindungsklassen enthält.

6. Verfahren gemäß Anspruch 5, dadurch gekennzeichnet, daß das verwendete Lösungsmittelgemisch eines oder mehrere weniger polare Lösungsmittel enthält.

7. Verfahren gemäß einem der Ansprüche 1 bis 4, dadurch gekennzeichnet, daß man bei der Herstellung der diastereomeren Salze Ethylacetat, 2-Butanon oder Aceton als Lösungsmittel verwendet, ggf. in Kombination mit einem weniger polaren Lösungsmittel wie tert. Butylmethylether, Diisopropylether, Toluol oder Butylacetat.

8. Verfahren gemäß einem der Ansprüche 1 bis 7, dadurch gekennzeichnet, daß man bei der Freisetzung der racemischen Imino-a-carbonsäureester der Formeln la und Ib aus ihren Salzen mit anorganischen Säuren tert. Butylmethylether oder Diisopropylether als Lösungsmittel verwendet.

9. Verfahren gemäß einem der Ansprüche 1 bis 8, dadurch gekennzeichnet, daß man das kristalline, diastereomere Salz ohne weitere Reinigung zerlegt.

10. Verfahren gemäß einem der Ansprüche 1 bis 7, dadurch gekennzeichnet, daß man das nach Anspruch 7 erhaltene diastereomere Salz durch Umkristallisieren, Umfällen oder Ausrühren reinigt, wobei die gleichen Lösungsmittel bzw. Lösungsmittelgemische wie im Anspruch 7 beschrieben, oder ein ähnliches Lösungsmittel oder Gemisch verwendet werden.

11. Verfahren gemäß einem der Ansprüche 1 bis 7, dadurch gekennzeichnet, daß man die diastereomeren Salze von Verbindungen der Formel la bzw. Ib mit Verbindungen der Formel II mittels wäßrigem Alkalihydroxyd zerlegt und die optisch reinen Basen der Formel la bzw. Ib in einem mit Wasser nicht mischbaren, organischen Lösungsmittel aufnimmt.

12. Verfahren gemäß einem der Ansprüche 1 bis 7, dadurch gekennzeichnet, daß man die optisch reinen Basen direkt aus dem organischen Lösungsmittel nach Anspruch 11 mit einer anorganischen Säure als Salze ausfällt und isoliert.

13. Diastereomeres Salz aus einem bicyclischen Imino-α-carbonsäureester der Formel la oder Ib mit einer optisch aktiven O,O-Diacylwein- säure der Formel II, welche wie im Anspruch 1 definiert sind.

14. Diastereomeres Salz gemäß Anspruch 13, dadurch gekennzeichnet, daß R² für Benzoyl steht.

15. Diastereomeres Salz gemäß Anspruch 13 oder 14, dadurch gekennzeichnet, daß R¹ für Benzyl oder tert.-Butyl steht.

16. Diastereomeres Salz gemäß einem der Ansprüche 13 bis 15, dadurch gekennzeichnet, daß A, B und C wie im Anspruch 3 definiert sind.

17. Diastereomeres Salz gemäß Anspruch 16, dadurch gekennzeichnet, daß die Verbindungen der Formel la und Ib die im Anspruch 2 definierte Konfiguration haben.

## Patentansprüche (Patentansprüche für folgende(n) Vertragsstaat(en) : AT)

1. Verfahren zur Auftrennung racemischer Gemische bicyclischer lmino-a-carbonsäureester in die Komponenten der Formel la und Ib 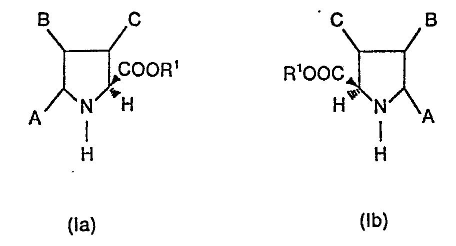 in welcher
R¹ für Alkyl mit 1 bis 6 C-Atomen, Cycloalkyl mit 4 bis 8 C-Atomen oder Aralkyl mit 7 bis 13 C-Atomen steht,
a) A Wasserstoff bedeutet und
B und C gemeinsam eine Kette der Formel -[CH₂]ₙ- mit n = 3, 4, 5 oder 6 oder eine Kette der Formel -[CH₂]ₚ-CH=CH-[CH₂]_{q}- mit (p + q) = 1, 2, 3 oder 4 bilden oder
b) C Wasserstoff bedeutet und
A und B gemeinsam eine der vorstehend unter a) definierten Ketten bilden
durch Kristallisation diastereomerer Salze, dadurch gekennzeichnet, daß man die Salze der racemischen Ester der Formel la und Ib mit optisch aktiven O,O-Diacylweinsäuren der Formel II 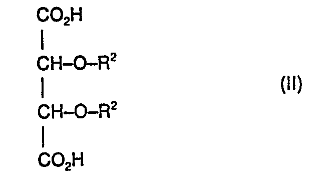 worin R² (C₁-C₆)-Alkanoyl oder (C₇-C₁₁)-Aroyl bedeutet, in einem Ester, einem Keton, einem Ether, einem niedrigen Alkohol, einem Nitril oder einem halogenierten Kohlenwasserstoff, oder einem Lösungsmittelgemisch, das eines oder mehrere Lösungsmittel aus den aufgezählten Verbindungsklassen enthält, herstellt, eines der beiden diastereomeren Salze kristallisiert, dieses diastereomere Salz ggf. durch Umkristallisieren, Umlösen oder Ausrühren reinigt und schließlich durch Zusatz von Base in die reinen Enantiomeren der Formeln la bzw. Ib zerlegt und diese direkt weiterverwendet oder durch Salzbildung mit Mineralsäure in eine lagerfähige Form bringt.

2. Verfahren nach Anspruch 1, dadurch gekennzeichnet, daß man die Salze von Estern der Formeln la bzw. Ib, in welchen die beiden Brückenkopf-Wasserstoffatome cis-konfiguriert sind und die COOR-Gruppe endo-ständig zum bicyclischen Ringsystem orientiert sind, mit Säuren der Formel II herstellt und kristallisiert.

3. Verfahren nach Anspruch 1 oder 2, dadurch gekennzeichnet, daß A und B gemeinsam für -[CH₂]ₙ- mit n = 3 oder 4 stehen und C Wasserstoff bedeutet.

4. Verfahren gemäß einem der Ansprüche 1 bis 3, dadurch gekennzeichnet, daß man die diastereomeren Salze in einem Ester, einem Keton, einem Ether, einem niedrigen Alkohol, einem Nitril oder einem halogenierten Kohlenwasserstoff herstellt.

5. Verfahren gemäß einem der Ansprüche 1 bis 4, dadurch gekennzeichnet, daß man die diastereomeren Salze in einem Lösungsmittelgemisch herstellt, das eines oder mehrere Lösungsmittel aus der Reihe der im Anspruch 4 aufgezählten Verbindungsklassen enthält.

6. Verfahren gemäß Anspruch 5, dadurch gekennzeichnet, daß das verwendete Lösungsmittelgemisch eines oder mehrere weniger polare Lösungsmittel enthält.

7. Verfahren gemäß einem der Ansprüche 1 bis 4, dadurch gekennzeichnet, daß man bei der Herstellung der diastereomeren Salze Ethylacetat, 2-Butanon oder Aceton als Lösungsmittel verwendet, ggf. in Kombination mit einem weniger polaren Lösungsmittel wie tert. Butylmethylether, Diisopropylether, Toluol oder Butylacetat.

8. Verfahren gemäß einem der Ansprüche 1 bis 7, dadurch gekennzeichnet, daß man bei der Freisetzung der racemischen Imino-a-carbonsäureester der Formeln la und Ib aus ihren Salzen mit anorganischen Säuren tert. Butylmethylether oder Diisopropylether als Lösungsmittel verwendet.

9. Verfahren gemäß einem der Ansprüche 1 bis 8, dadurch gekennzeichnet, daß man das kristalline, diastereomere Salz ohne weitere Reinigung zerlegt.

10. Verfahren gemäß einem der Ansprüche 1 bis 7, dadurch gekennzeichnet, daß man das nach Anspruch 7 erhaltene diastereomere Salz durch Umkristallisieren, Umfällen oder Ausrühren reinigt, wobei die gleichen Lösungsmittel bzw. Lösungsmittelgemische wie im Anspruch 7 beschrieben, oder ein ähnliches Lösungsmittel oder Gemisch verwendet werden.

11. Verfahren gemäß einem der Ansprüche 1 bis 7, dadurch gekennzeichnet, daß man die diastereomeren Salze von Verbindungen der Formel la bzw. Ib mit Verbindungen der Formel II mittels wäßrigem Alkalihydroxyd zerlegt und die optisch reinen Basen der Formel la bzw. Ib in einem mit Wasser nicht mischbaren, organischen Lösungsmittel aufnimmt.

12. Verfahren gemäß einem der Ansprüche 1 bis 7, dadurch gekennzeichnet, daß man die optisch reinen Basen direkt aus dem organischen Lösungsmittel nach Anspruch 11 mit einer anorganischen Säure als Salze ausfällt und isoliert.

13. Verfahren zur Herstellung eines diastereomeren Salzes aus einem bicyclischen Imino-a-carbonsäureester der Formel la oder Ib mit einer optisch aktiven O,O-Diacylweinsäure der Formel II, welche wie im Anspruch 1 definiert sind, dadurch gekennzeichnet, daß man das Salz der racemischen Ester der Formel la und Ib mit einer optischen aktiven O,O-Diacylweinsäure der Formel II in einem Ester, einem Keton, einem Ether, einem niedrigen Alkohol, einem Nitril oder einem halogenierten Kohlenwasserstoff oder einem Lösungsmittelgemisch, das eines oder mehrere Lösungsmittel aus den aufgezählten Verbindungsklassen enthält, herstellt, eines der beiden diastereomeren Salze kristallisiert, dieses diastereomere Salz ggfs. durch Umkristallisieren, Umlösen oder Ausrühren reinigt.

14. Verfahren gemäß Anspruch 13, dadurch gekennzeichnet, daß R² für Benzoyl steht.

15. Verfahren gemäß Anspruch 13 oder 14, dadurch gekennzeichnet, daß R¹ für Benzyl oder tert.-Butyl steht.

16. Verfahren gemäß einem der Ansprüche 13 bis 15, dadurch gekennzeichnet, daß A, B und C wie im Anspruch 3 definiert sind.

17. Verfahren gemäß Anspruch 16, dadurch gekennzeichnet, daß die Verbindungen der Formel la und Ib die im Anspruch 2 definierte Konfiguration haben.

## Claims (Claims for the following Contracting State(s) : BE, CH, DE, FR, GB, IT, LI, LU, NL, SE)

1. A process for resolving racemic mixtures of bicyclic imino-a-carboxylic acid esters into the components of the formulae la and Ib 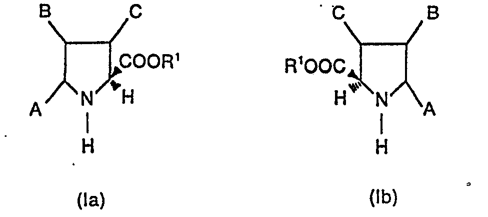 in which
R¹ stands for alkyl of 1 to 6 carbon atoms, cycloalkyl of 4 to 8 carbon atoms, or aralkyl of 7 to 13 carbon atoms,
a) A denotes hydrogen and
B and C together form a chain of the formula -[CH₂]ₙ- with n = 3, 4, 5 or 6 or a chain of the formula-[CH₂]ₚ-CH=CH-[CH₂]q- with (p + q) = 1, 2. 3 or 4 or
b) C denotes hydrogen and
A and B together form one of the chains defined above under a)
by crystallizing diastereoisomeric salts, which comprises preparing the salts of the racemic esters of the formulae la and Ib with optically active O,O-diacyltartaric acids of the formula II 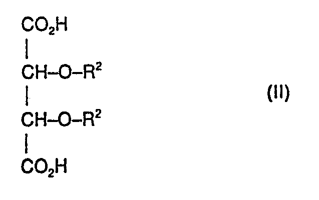 in which R² denotes (C₁-C₆)-alkanoyl or (C₇-C₁₁)-aroyl, in an ester, a ketone, an ether, a lower alcohol, a nitrile, or a halogenated hydrocarbon or in a solvent mixture which contains one or more solvents from the compound classes listed, crystallizing one of the two diastereoisomeric salts, if desired purifying this diastereoisomeric salt by recrystallization, reprecipitation or trituration, finally cleaving the salt into the pure enantiomers of the formulae la or Ib by adding base and directly using these enantiomers or bringing them into a storable form by salt formation with mineral acid.

2. The process as claimed in claim 1, characterized in that the salts of esters of the formulae la and lb in which the two bridgehead hydrogen atoms are in the cis configuration and the COOR group is oriented in the endo-position relative to the bicyclic ring system are prepared with acids of the formula II and crystallized.

3. The process as claimed in claim 1 or 2, characterized in that A and B together stand for -[CH₂]ₙ- with n = 3 or 4 and C denotes hydrogen.

4. The process according to any one of claims 1 to 3, characterized in that the diastereoisomeric salts are prepared in an ester, a ketone, an ether, a low alcohol, a nitrile or a halogenated hydrocarbon.

5. The process according to any one of claims 1 to 4, characterized in that the diastereoisomeric salts are prepared in a solvent mixture which contains one or more solvents from the series of the compound classes listed in claim 4.

6. The process as claimed in claim 5, characterized in that the solvent mixture used contains one or more less polar solvents.

7. The process as claimed in any one of claims 1 to 4, characterized in that the solvent used in the preparation of the diastereoisomeric salts is ethyl acetate, 2-butanone or acetone, if desired combined with a less polar solvent such as tert.-butyl methyl ether, diisopropyl ether, toluene or butyl acetate.

8. The process as claimed in any one of claims 1 to 7, characterized in that the solvent used in the liberation of the racemic imino-α-carboxylic acid esters of the formulae la and Ib from their salts with inorganic acids is tert.-butyl methyl ether or diisopropyl ether.

9. The process as claimed in any one of claims 1 to 8, characterized in that the crystalline diastereoisomeric salt is cleaved without further purification.

10. The process as claimed in any one of claims 1 to 7, characterized in that the diastereoisomeric salt obtained in claim 7 is purified by recrystallization, reprecipitation or trituration, the same solvents or solvent mixtures as described in claim 7 or a similar solvent or mixture being used.

11. The process as claimed in any one of claims 1 to 7, characterized in that the diastereoisomeric salts of compounds of the formula la or Ib with compounds of the formula II are cleaved by means of aqueous alkali metal hydroxide and the optically pure bases of the formula la or Ib are taken up in an organic solvent which is immiscible with water.

12. The process as claimed in any one of claims 1 to 7, characterized in that an inorganic acid is used to precipitate the optically pure bases directly from the organic solvent of claim 11 in salt form, and the salts are isolated.

13. A diastereoisomeric salt of a bicyclic imino-a-carboxylic acid ester of the formula la or Ib with an optically active O,O-diacyltartaric acid of the formula II which are as defined as in claim 1.

14. A diastereoisomeric salt as claimed in claim 13, characterized in that R² stands for benzoyl.

15. A diastereoisomeric salt as claimed in claim 13 or 14, characterized in that R¹ stands for benzyl or tert.-butyl.

16. A diastereoisomeric salt as claimed in any one of claims 13 to 15, characterized in that A, B and C are as defined in claim 3.

17. A diastereoisomeric salt as claimed in claim

. 16, characterized in that the compounds of the formulae la and Ib have the configuration defined in claim 2.

## Claims (Claims for the following Contracting State(s) : AT)

1. A process for resolving racemic mixtures of bicyclic imino-a-carboxylic acid esters into the components of the formulae la and Ib 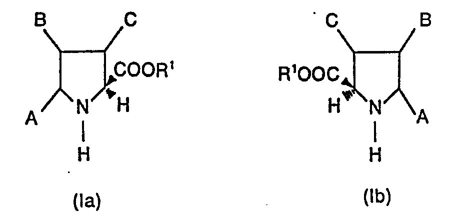 in which
R'stands for alkyl of 1 to 6 carbon atoms, cycloalkyl of 4 to 8 carbon atoms, or aralkyl of 7 to 13 carbon atoms,
a) A denotes hydrogen and
B and C together form a chain of the formula -[CH2]ₙ- with n = 3, 4, 5 or 6 or a chain of the formula-[CH₂]ₚ-CH=CH-[CH₂]_{q}- with (p + q) = 1, 2, 3 or 4 or
b) C denotes hydrogen and
A and B together form one of the chains defined above under a)
by crystallizing diastereoisomeric salts, which comprises preparing the salts of the racemic esters of the formulae la and Ib with optically active O,O-diacyltartaric acids of the formula II 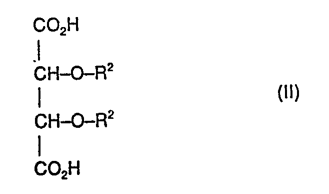 in which R² denotes (C₁-C₆)-alkanoyl or (C₇-C₁₁)-aroyl, in an ester, a ketone, an ether, a lower alcohol, a nitrile, or a halogenated hydrocarbon or in a solvent mixture which contains one or more solvents from the compound classes listed, crystallizing one of the two diastereoisomeric salts, if desired purifying this diastereoisomeric salt by recrystallization, reprecipitation or trituration, finally cleaving the salt into the pure enantiomers of the formulae la or Ib by adding base and directly using these enantiomers or bringing them into a storable form by salt formation with mineral acid.

2. The process as claimed in claim 1, characterized in that the salts of esters of the formulae la and Ib in which the two bridgehead hydrogen atoms are in the cis configuration and the COOR group is oriented in the endo-position relative to the bicyclic ring system are prepared with acids of the formula II and crystallized.

3. The process as claimed in claim 1 or 2, characterized in that A and B together stand for -[CH₂]ₙ- with n = 3 or 4 and C denotes hydrogen.

4. The process according to any one of claims 1 to 3, characterized in that the diastereoisomeric salts are prepared in an ester, a ketone, an ether, a low alcohol, a nitrile or a halogenated hydrocarbon.

5. The process according to any one of claims 1 to 4, characterized in that the diastereoisomeric salts are prepared in a solvent mixture which contains one or more solvents from the series of the compound classes listed in claim 4.

6. The process as claimed in claim 5, characterized in that the solvent mixture used contains one or more less polar solvents.

7. The process as claimed in any one of claims 1 to 4, characterized in that the solvent used in the preparation of the diastereoisomeric salts is ethyl acetate, 2-butanone or acetone, if desired combined with a less polar solvent such as tert.-butyl methyl ether, diisopropyl ether, toluene or butyl acetate.

8. The process as claimed in any one of claims 1 to 7, characterized in that the solvent used in the liberation of the racemic imino-a-carboxylic acid esters of the formulae la and Ib from their salts with inorganic acids is tert.-butyl methyl ether or diisopropyl ether.

9. The process as claimed in any one of claims 1 to 8, characterized in that the crystalline diastereoisomeric salt is cleaved without further purification.

10. The process as claimed in any one of claims 1 to 7, characterized in that the diastereoisomeric salt obtained in claim 7 is purified by recrystallization, reprecipitation or trituration, the same solvents or solvent mixtures as described in claim 7 or a similar solvent or mixture being used.

11. The process as claimed in anyone of claims 1 to 7, characterized in that the diastereoisomeric salts of compounds of the formula la or Ib with compounds of the formula II are cleaved by means of aqueous alkali metal hydroxide and the optically pure bases of the formula la or Ib are taken up in an organic solvent which is immiscible with water.

12. The process as claimed in any one of claims 1 to 7, characterized in that an inorganic acid is used to precipitate the optically pure bases directly from the organic solvent of claim 11 in salt form, and the salts are isolated.

13. A process for preparing a diastereoisomeric salt of a bicyclic imino-a-carboxylic acid ester of the formula la or Ib with an optically active 0,0- diacyltartaric acid of the formula II, which are defined as in claim 1, which comprises preparing the salt of the racemic ester of the formulae la and Ib with an optically active O,O-diacyltartaric acid of the formula II in an ester, a ketone, an ether, a lower alcohol, a nitrile or a halogenated hydrocarbon or in a solvent mixture which contains one or more solvents from the compound classes listed, crystallizing one of the two diastereoisomeric salts and, if desired purifying this diastereoisomeric salt by recrystallization, reprecipitation or trituration.

14. The process as claimed in claim 13, characterized in that R² stands for benzoyl.

15. The process as claimed in claim 13 or 14, characterized in that R¹ stands for benzyl or tert.- butyl.

16. The process as claimed in any one of claims 13 to 15, characterized in that A, B and C are as defined in claim 3.

17. The process as claimed in claim 16, characterized in that the compounds of the formulae la and Ib have the configuration defined in claim 2.

## Revendications (Revendications pour l'(les) Etat(s) contractant(s) suivant(s) : BE, CH, DE, FR, GB, IT, LI, LU, NL, SE)

1. Procédé pour le dédoublement de mélanges racémiques d'esters imino-a-carboxyliques bicycliques en les composants de formules la et Ib 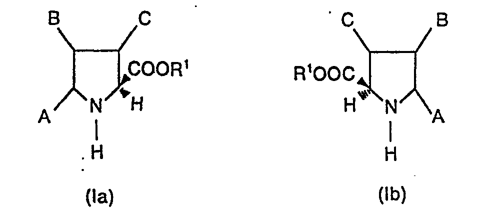 dans lesquelles
R¹ représente un groupe alkyle ayant de 1 à 6 atomes de carbone, un groupe cycloalkyle ayant de 4 à 8 atomes de carbone ou un groupe aralkyle ayant de 7 à 13 atomes de carbone,
a) A représente un atome d'hydrogène et
B et C forment ensemble une chaîne de formule -[CH₂]ₙ- avec n = 3, 4, 5 ou 6, ou une chaîne de formule -[CH₂]ₚ-CH =CH-[CH₂]_{q}- avec (p + q) = 1, 2, 3 ou 4, ou
b) C représente un atome d'hydrogène et
A et B forment ensemble l'une des chaînes définies ci-dessus en a),
par cristallisation de sels diastéréoisomères, caractérisé en ce que l'on prépare les sels des esters racémiques de formules la et Ib avec des acides O,O-diacyltartriques optiquement actifs de formule Il 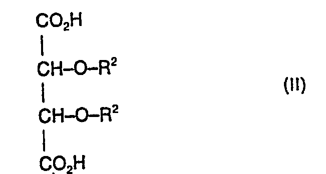 dans laquelle R² représente un groupe alcanoyle en C₁-C₆ ou aroyle en C₇-C₁₁, dans un ester, une cétone, un éther, un alcool inférieur, un nitrile ou un hydrocarbure halogéné, ou dans un mélange de solvants qui contient un ou plusieurs solvants choisis dans les catégories de composés énumérés, on fait cristalliser l'un des deux sels diastéréoisomères, on purifie éventuellement ce sel diastéréoisomère par recristallisation, redissolution ou extraction sous agitation, et on le décompose enfin, par addition d'une base, en les énan- tiomères purs de formule la ou Ib, puis on soumet directement ceux-ci à l'utilisation ultérieure, ou on les met sous une forme stable au stockage par salification avec un acide minéral.

2. Procédé selon la revendication 1, caractérisé en ce que l'on prépare avec des acides de formule II et cristallise les sels d'esters de formules la et Ib, dans lesquels les deux atomes d'hydrogène de tête de pont sont en configuration cis et le groupe COOR est orienté en position endo par rapport au système nucléaire bicyclique.

3. Procédé selon la revendication 1 ou 2, caractérisé en ce que A et B forment ensemble un groupe -[CH2]ₙ- avec n = 3 ou 4, et C représente un atome d'hydrogène.

4. Procédé selon l'une des revendications 1 à 3, caractérisé en ce que l'on prépare les sels diastéréoisomères dans un ester, une cétone, un éther, un alcool inférieur, un nitrile ou un hydrocarbure halogéné.

5. Procédé selon l'une des revendications 1 à 4, caractérisé en ce que l'on prépare les sels diastéréoisomères dans un mélange de solvants qui contient un ou plusieurs solvants choisis dans les catégories de composés énumérées dans la ré- vendication 4.

6. Procédé selon la revendication 5, caractérisé en ce que le mélange de solvants utilisé contient un ou plusieurs solvants faiblement polaires.

7. Procédé selon l'une des revendications 1 à 4, caractérisé en ce que, dans la préparation des sels diastéréoisomères, on utilise en tant que solvant l'acétate d'éthyle, la butanone-2 ou l'acétone, éventuellement en association avec un solvant faiblement polaire tel que l'éther tert-butylméthylique, l'éther diisopropylique, le toluène ou l'acétate de butyle.

8. Procédé selon l'une des revendications 1 à 7, caractérisé en ce que, dans la libération des esters imino-a-carboxyliques racémiques de formules la et Ib à partir de leurs sels avec des acides minéraux, on utilise en tant que solvant l'éther tert-butylméthylique ou l'éther diisopropylique.

9. Procédé selon l'une des revendications 1 à 8, caractérisé en ce que l'on décompose le sel diastéréoisomère cristallin sans le purifier davantage.

10. Procédé selon l'une des revendications 1 à 7, caractérisé en ce que l'on purifie par recristallisation, reprécipitation ou extraction sous agitation le sel diastéréoisomère obtenu selon la revendication 7, en utilisant les mêmes solvants ou mélanges de solvants que ceux décrits dans la revendication 7, ou un solvant ou mélange de solvants similaire.

11. Procédé selon l'une des revendications 1 à 7, caractérisé en ce que l'on décompose au moyen d'une solution aqueuse d'un hydroxyde alcalin les sels diastéréoisomères de composés de formule la ou Ib avec des composés de formule Il, et on reprend dans un solvant organique non miscible à l'eau les bases de formule la ou Ib optiquement pures.

12. Procédé selon l'une des revendications 1 à 7, caractérisé en ce que l'on fait précipiter sous forme de sels avec un acide minéral et isole directement dans le solvant organique selon la revendication 11 les bases optiquement pures.

13. Sel diastéréoisomère à partir d'un ester imino-a-carboxylique bicyclique de formule la ou Ib avec un acide O,O-diacyltartrique de formule Il optiquement actif, qui sont définis dans la revendication 1.

14. Sel diastéréoisomère selon la revendication 13, caractérisé en ce que R² représente le groupe benzoyle.

15. Sel diastéréoisomère selon la revendication 13 ou 14, caractérisé en ce que R¹ représente le groupe benzyle ou tert-butyle.

16. Sel diastéréoisomère selon l'une des revendications 13 à 15, caractérisé en ce que A, B et C sont définis dans la revendication 3.

17. Sel diastéréoisomère selon la revendication 16, caractérisé en ce que les composés de formules la et Ib ont la configuration définie dans la revendication 2.

## Revendications (Revendications pour l'(les) Etat(s) contractant(s) suivant(s) : AT)

1. Procédé pour le dédoublement de mélanges racémiques d'esters imino-a-carboxyliques bicycliques en les composants de formules la et Ib 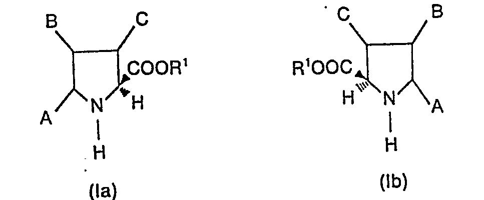 dans lesquelles
R¹ représente un groupe alkyle ayant de 1 à 6 atomes de carbone, un groupe cycloalkyle ayant de 4 à 8 atomes de carbone ou un groupe aralkyle ayant de 7 à 13 atomes de carbone,
a) A représente un atome d'hydrogène et
B et C forment ensemble une chaîne de formule -[CH₂]ₙ- avec n = 3, 4, 5 ou 6, ou une chaîne de formule-[CH₂]p-CH=CH-[CH₂]_{q}- avec (p + q) = 1, 2, 3 ou 4, ou
b) C représente un atome d'hydrogène et
A et B forment ensemble l'une des chaînes définies ci-dessus en a),
par cristallisation de sels diastéréoisomères, caractérisé en ce que l'on prépare les sels des esters racémiques de formules la et Ib avec des acides O,O-diacyltartriques optiquement actifs de formule II 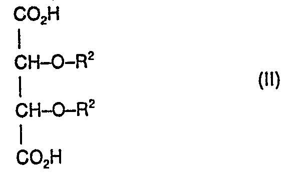 dans laquelle R² représente un groupe alcanoyle en C₁-C₆ ou aroyle en C₇-C₁₁, dans un ester, une cétone, un éther, un alcool inférieur, un nitrile ou un hydrocarbure halogéné, ou dans un mélange de solvants qui contient un ou plusieurs solvants choisis dans les catégories de composés énumérés, on fait cristalliser l'un des deux sels diastéréoisomères, on purifie éventuellement ce sel diastéréoisomère par recristallisation, redissolution ou extraction sous agitation, et on le décompose enfin, par addition d'une base, en les énantiomè- res purs de formule la ou Ib, puis on soumet directement ceux-ci à l'utilisation ultérieure, ou on les met sous une forme stable au stockage par salification avec un acide minéral.

2. Procédé selon la revendication 1, caractérisé en ce que l'on prépare avec des acides de formule Il et cristallise les sels d'esters de formules la et Ib, dans lesquels les deux atomes d'hydrogène de tête de pont sont en configuration cis et le groupe COOR est orienté en position endo par rapport au système nucléaire bicyclique.

3. Procédé selon la revendication 1 ou 2, caractérisé en ce que A et B forment ensemble un groupe -[CH₂]ₙ- avec n = 3 ou 4, et C représente un atome d'hydrogène.

4. Procédé selon l'une des revendications 1 à 3, caractérisé en ce que l'on prépare les sels diastéréoisomères dans un ester, une cétone, un éther, un alcool inférieur, un nitrile ou un hydrocarbure halogéné.

5. Procédé selon l'une des revendications 1 à 4, caractérisé en ce que l'on prépare les sels diastéréoisomères dans un mélange de solvants qui contient un ou plusieurs solvants choisis dans les catégories de composés énumérées dans la revendication 4.

6. Procédé selon la revendication 5, caractérisé en ce que le mélange de solvants utilisé contient un ou plusieurs solvants faiblement polaires.

7. Procédé selon l'une des revendications 1 à 4, caractérisé en ce que, dans la préparation des sels diastéréoisomères, on utilise en tant que solvant l'acétate d'éthyle, la butanone-2 ou l'acétone, éventuellement en association avec un solvant faiblement polaire tel que l'éther tert-butylméthylique, l'éther diisopropylique, le toluène ou l'acétate de butyle.

8. Procédé selon l'une des revendications 1 à 7, caractérisé en ce que, dans la libération des esters imino-a-carboxyliques racémiques de formules la et lb à partir de leurs sels avec des acides minéraux, on utilise en tant que solvant l'éther tert-butylméthylique ou l'éther diisopropylique.

9. Procédé selon l'une des revendications 1 à 8, caractérisé en ce que l'on décompose le sel diastéréoisomère cristallin sans le purifier davantage.

10. Procédé selon l'une des revendications 1 à 7, caractérisé en ce que l'on purifie par recristallisation, reprécipitation ou extraction sous agitation le sel diastéréoisomère obtenu selon la revendication 7, en utilisant les mêmes solvants ou mélanges de solvants que ceux décrits dans la revendication 7, ou un solvant ou mélange de solvants similaire.

11. Procédé selon l'une des revendications 1 à 7, caractérisé en ce que l'on décompose au moyen d'une solution aqueuse d'hydroxyde alcalin les sels diastéréoisomères de composés de formule la ou Ib avec des composés de formule II, et on reprend dans un solvant organique non miscible à l'eau les bases de formule la ou Ib optiquement pures.

12. Procédé selon l'une des revendications 1 à 7, caractérisé en ce que l'on fait précipiter sous forme de sels avec un acide minéral et isole directement dans le solvant organique selon la revendication 11 les bases optiquement pures.

13. Procédé pour la préparation d'un sel diastéréoisomère à partir d'un ester imino-a-carboxylique bicyclique de formule la ou Ib avec un acide O,O-diacyltartrique de formule Il optiquement actif, qui sont tels que définis dans la revendication 1, caractérisé en ce que l'on prépare le sel de l'ester racémique de formules la et Ib avec un acide O,O-diacyltartrique de formule Il optiquement actif, dans un ester, une cétone, un éther, un alcool inférieur, un nitrile ou un hydrocarbure halogéné ou dans un mélange de solvants qui contient un ou plusieurs solvants choisis dans les catégories de composés énumérés, on cristallise l'un des deux sels diastéréoisomères et éventuellement on purifie ce sel diastéréoisomère par recristallisation, redissolution ou extraction sous agitation.

14. Procédé selon la revendication 13, caractérisé en ce que R² représente le groupe benzoyle.

15. Procédé selon la revendication 13 ou 14, caractérisé en ce que R¹ représente le groupe benzyle ou tert-butyle.

16. Procédé selon l'une des revendications 13 à 15, caractérisé en ce que A, B et C sont définis dans la revendication 3.

17. Procédé selon la revendication 16, caractérisé en ce que les composés de formules la et lb ont la configuration définie dans la revendication 2.
